# EUROPEAN PATENT APPLICATION

(11) **EP 0 787 580 A1**
(43) Date of publication of application: **06.08.1997**
(21) Application number: 97100568.1
(22) Date of filing: 16.01.1997
(51) Int. Cl.: B32B 27/08, A61L 25/00

(54) **Gas-barrier multi-layer thermoplastic film and containers for medical use made therefrom**

(30) Priority: 30.01.1996 EP 96101242
(71) Applicant: W.R. Grace & Co.-Conn., New York New York 10036 (US)
(72) Inventor: Buongiorno, Livio, 20090 Trezzano S/Naviglio (Milan) (IT); Perego, Vittorio, 21052 Busto Arsizio (Varese) (IT)
(74) Representative: De Carli, Elda

(57) **Abstract**

Multilayer gas- and odour-barrier films are formed comprising
i) a core gas- and odour-barrier layer
ii) a first exterior sealing layer
iii) a second exterior abuse and skin contact layer
   and
iv) at least one intermediate layer positioned between the core barrier layer and at least one of the exterior layers ii) and iii), comprising an olefinic elastomer optionally blended with an ethylene homopolymer and/or copolymer.

Such films are suitable for the manufacture of containers for medical applications, and particularly of bags or pouches for drainage or collection of human excretion products.

## Description

The present invention relates to a gas-barrier multi-layer thermoplastic film with improved properties of noiselessness as well as of softness and flexibility, and to containers and bags intended for human drainage in medical applications.

For certain medical applications, containers, such as bags or pouches, are required for use in contact with the skin, under the patient's garments, for collecting excretion products from patients whose excretive apparatus has been deviated or reconstructed surgically following traumatic or pathological events. These containers or bags are generally referred to as "ostomy" pouches.

Films suitable for such a specific end use should meet a number of requirements : they must be gas- and odour-barrier; have mechanical properties that afford a good degree of protection against wear, abrasion and puncturing; possess quietness features that render the sound emission, particularly when the patients moves around, almost impossible to be perceived by a human ear; and softness and "feel" features that make them suited to applications involving compatibility with human skin. Furthermore they should be sealable, e.g. heat or RF sealable, so that no leakage, even minimal, occurs at the seal.

Currently available from a number of manufacturers are excretion product collecting containers which meet some of the requirements set forth above. In particular commercially available ostomy pouches, such as those with a PVDC gas- and odour-barrier core layer and outer layers of e.g. ethylene-vinyl acetate copolymers, typically have good mechanical and gas- and odour-barrier properties while they are generally poor in noiselessness and softness so that the patient is never allowed to at least occasionally "forget" his/her disabled condition, with obvious disturbing consequences of a psychological nature.

It is known to improve the softness and noiselessness of films for medical use by employing blends of ethylene homopolymers and/or copolymers and olefinic elastomers either in mono-layer structures e.g. for medical gloves, surgical films and table covers or in multi-layer structures as the outer liquid impervious layers e.g. of liquid absorbent products such as disposable diapers, sanitary napkins, etc..

The advantage of using these blends as the outer skin layer of a multi-layer structure resides in the fact that they work as liquid impervious layer, and provide a high degree of comfort as the noise is substantially eliminated.

Examples of blends recommended for use in the medical field are for instance those commercialised by Exxon Chemicals as Escorene EA 001 and EA 002, that comprise about 40-50 parts by weight of an ethylene-vinyl acetate copolymer, about 35-45 parts by weight of an ethylene rubber and about 5-10 parts by weight of a hydrocarbon processing oil. However, these blends are in general characterised by a very low Vicat softening point (for those commercialised by Exxon, the Vsp is in the range 40-55 °C) and therefore their use as the outer skin-contact layer of an ostomy pouch is clearly unacceptable as the temperature attained by the material in contact with the body would be very close to its Vicat softening temperature with obvious risks of deformation of the pouch itself.

BE-A-899,649 describes films particularly suitable for use in ostomy applications wherein the outer skin layers comprise a mixture of 60-40 wt. % of such blends comprising 35-70 wt. % of an ethylene-vinyl acetate copolymer and 35-70 wt. % of an elastomeric copolymer of ethylene and propylene with 40-60 wt. % of an ethylene-vinyl acetate copolymer.

It has now been found that it is possible to obtain a film having high mechanical and gas- and odour-barrier properties and remarkable softness and noiselessness features that can suitable be employed for ostomy applications by providing a multi-layer film comprising a core gas-barrier and two exterior layers characterised in that it comprises at least an intermediate layer comprising an olefinic elastomer optionally blended with an ethylene homo- or co-polymer.

A first object of the present invention is therefore a film which, in addition to very good mechanical and odour-barrier properties, has improved quietness and softness features.

A second object of the present invention is a bag or container intended in particular for human draining and collecting excretion products from patients having a deviated reconstructed excretive apparatus which exhibits high noiselessness and human skin compatibility features.

### SUMMARY OF THE INVENTION

The first object of the present invention is met by a multi-layer gas- and odour-barrier film comprising
i) a core gas- and odour-barrier layer
ii) a first exterior sealing layer
iii) a second exterior abuse and skin contact layer
   and
iv) at least one intermediate layer positioned between the core barrier layer and at least one of the exterior layers ii) and iii), comprising an olefinic elastomer optionally blended with an ethylene homopolymer and/or copolymer.

As used herein, the term "film" is used in a generic sense to include any flexible plastic web, regardless of whether it is film or sheet. Preferably, films of use in the present invention have a thickness of 250 µ or less, more preferably of 150 µ or less.

As used herein, the phrases "inner layer", "internal layer", or "intermediate layer" refer to any film layer having both of its principal surfaces directly adhered to another layer of the film.

As used herein, the term "core", and the phrase "core layer" refer to any internal film layer which has a primary function other than serving as an adhesive or compatibilizer for adhering two layers to one another.

As used herein, the term "barrier", and the phrase "barrier layer", as applied to films and/or film layers, is used with reference to the ability of a film or film layer to serve as a barrier to gasses and odours.

As used herein, the phrase "outer layer" or "exterior layer" refers to any film layer having less than two of its principal surfaces directly adhered to another layer of the film. In multi-layer films, there are two outer layers, each of which has a principal surface adhered to only one other layer of the multilayer film.

As used herein, the phrases "seal layer", "sealing layer", "heat seal layer", and "sealant layer", refer to an exterior film layer involved in the sealing of the film to itself, another film layer of the same or another film, and/or another article which is not a film.

As used herein, the term "seal" refers to any seal of a first region of a film surface to a second region of a film surface, wherein the seal is formed by heating the regions to at least their respective seal initiation temperatures. The heating can be performed by any one or more of a wide variety of manners, such as using a heated bar, hot air, infrared radiation, etc.

As used herein, the phrase "directly adhered", as applied to film layers, is defined as adhesion of the subject film layer to the object film layer, without a tie layer, adhesive, or other layer therebetween. In contrast, as used herein, the word "between", as applied to a film layer expressed as being between two other specified layers, includes both direct adherence of the subject layer to the two other layers it is between, as well as a lack of direct adherence to either or both of the two other layers the subject layer is between, i.e., one or more additional layers can be imposed between the subject layer and one or both the layers the subject layer is between.

As used herein, the term "polymer" refers to the product of a polymerization reaction, and is inclusive of homopolymers, copolymers, terpolymers, etc.

As used herein, the term "polyolefin" means a thermoplastic homo-, co- or ter-polymer derived from simple olefins, e.g. ethylene, propylene and higher unsaturated aliphatic monomer units, or the halogenated derivatives thereof, as well as the co- or ter-polymers of said simple olefins with co-monomers that are not themselves olefins, e.g. vinyl acetate, acrylic or methacrylic acids, salts, or esters, provided however that the olefin comonomer is present in a major amount. More specifically, included in the term polyolefin are homopolymers of olefins, copolymers of olefins, copolymers of an olefin and a non-olefinic comonomer copolymerizable with the olefin, such as vinyl monomers, and the like. Heterogeneous and homogeneous polymers are included. Specific examples include polyethylene homopolymers, poly-butene, propylene-α-olefin copolymers, ethylene-α-olefin copolymers, butene-α-olefin copolymers, ethylene-vinyl acetate copolymers, ethylene-ethyl acrylate copolymers, ethylene-butyl acrylate copolymers, ethylene-methyl acrylate copolymers, ethylene-acrylic acid copolymers, ethylene-methacrylic acid copolymers, ionomers, chlorinated polyethylene, etc.

As used herein, the phrase "heterogeneous polymer" refers to polymerization reaction products of relatively wide variation in molecular weight and relatively wide variation in composition distribution. Such polymers typically contain a relatively wide variety of chain lengths and comonomer percentages.

As used herein, the phrase "homogeneous polymer" refers to polymerization reaction products of relatively narrow molecular weight distribution and relatively narrow composition distribution. Homogeneous polymers exhibit a relatively even sequencing of comonomers within a chain, the mirroring of sequence distribution in all chains, and the similarity of length of all chains.

As used herein, the phrase "ethylene-α-olefin copolymer" refers to such heterogeneous materials as linear low density polyethylene (LLDPE), and very low and ultra low density polyethylene (VLDPE and ULDPE); and homogeneous polymers such as Ziegler-Natta-catalyzed homogenous polymers, for example TAFMER™ materials supplied by Mitsui Petrochemical, and metallocene-catalyzed homogenous polymers, such as EXACT™ materials supplied by Exxon, AFFINITY™ or ENGAGE™ resins supplied by Dow, or LUFLEXEN™ materials supplied by BASF.

As used herein the term "modified polyolefins" include modified polymers prepared by copolymerizing the homopolymer of the olefin or copolymer thereof with an unsaturated carboxylic acid, e.g., maleic acid, fumaric acid or the like, or a derivative thereof such as the anhydride, ester or metal salt or the like, or by incorporating into the olefin homopolymer or copolymer, an unsaturated carboxylic acid, e.g., maleic acid, fumaric acid or the like, or a derivative thereof such as the anhydride, ester or metal salt or the like.

As used herein, the phrase "tie layer" refers to any internal layer having the primary purpose of adhering two layers to one another. Tie layers generally comprise a non-polar or slightly polar polymer having a polar group grafted thereon; preferably, tie layers comprise at least one member selected from the group consisting of polyolefin and modified polyolefin, e.g. ethylene-vinyl acetate copolymer, modified ethylene-vinyl acetate copolymer, heterogeneous and homogeneous ethylene-α-olefin copolymer, and modified heterogeneous and homogeneous ethylene-α-olefin copolymer; more preferably, tie layers comprise at least one member selected from the group consisting of anhydride grafted linear low density polyethylene, anhydride grafted low density polyethylene, homogeneous ethylene-α-olefin copolymer, and anhydride grafted ethylene-vinyl acetate copolymer.

In a preferred embodiment of the present invention there are two intermediate layers iv), each one being positioned between the core gas-barrier layer i) and either one of the exterior layers ii) and iii).

In a most preferred embodiment the film is symmetrical.

Additional interior layers may be present, such as tie layers to improve cohesion between the different layers or moisture barrier layers if the gas- and odour-barrier material is sensible to moisture, etc.

In a more preferred embodiment however the film is a 5-layer film wherein two intermediate layers iv) are present, each one having one surface directly adhered to the opposite surfaces of the core barrier layer i) and the other surface directly adhered to the inner surfaces of the outer layers ii) and iii) respectively.

In another more preferred embodiment the film is a 7-layer film wherein two tie layers are present to adhere each of the intermediate layers iv) to the core barrier layer i).

The gas- and odour-barrier layer i) may comprise conventional materials, as normally suitable for this purpose, such as vinylidene chloride copolymers with a comonomer selected from vinyl chloride, acrylic esters, and acrylic acid (PVDC) or a blend thereof, a polyamide, an ethylene-vinyl alcohol copolymer, or a polyvinyl alcohol.

Preferably however the barrier layer i) will comprise an ethylene-vinyl alcohol copolymer or PVDC. More preferably it will comprise PVDC.

The thickness of the barrier layer can vary from about 3 to about 20 µ, typically ranging from about 8 to about 15 µ, and more preferably from about 9 to about 13 µ.

The sealing layer ii) will comprise a thermoplastic polyolefin. Preferably the sealing layer ii) is made of one or more thermoplastic polyolefins. Typically said thermoplastic polyolefin will be selected from ethylene homo- and co-polymers. Examples of suitable resins that can be used, either alone or blended with other resins, for said sealing layer are heterogeneous ethylene-α-olefins with a density below 0.925 g/cc, preferably below 0.915 g/cc, homogeneous ethylene-α-olefins with a density below 0.920 g/cc preferably below 0.910 g/cc, ethylene-vinyl acetate copolymers, ethylene-alkyl acrylate copolymers, e.g. ethylene-methyl acrylate copolymers, and ethylene-butyl acrylate copolymers, ethylene-alkyl methacrylate copolymers, ionomers and chlorinated polyethylene.

The thickness of the sealing layer ii) is typically higher than about 5 µ, preferably higher than about 9 µ, and even more preferably higher than about 13 µ.

The other exterior layer, layer iii), comprises a material that can suitably be put in contact with the skin, such as ethylene-vinyl acetate copolymers, ethylene-butyl acrylate copolymers, ethylene-methyl acrylate copolymers, heterogeneous and homogeneous ethylene-α-olefin copolymers, chlorinated polyolefins, etc. The melting point of the polymers used for the exterior layer iii) is preferably lower than 200 °C, even more preferably lower than 180 °C.

Olefinic elastomers suitable for the intermediate layers iv) may be ethylene-propylene elastomers (EPM) or ethylene-propylene-diene elastomers (EPDM) wherein the conjugated diene may be for example 1,4-hexadiene, dicyclopentadiene or ethylidene norbornene, elastomers such as polyisobutylenes (PIB), isobutylene-isoprene rubbers (IIR) and halogenated derivatives thereof, and thermoplastic rubbers such as styrene-butadiene-styrene block copolymers (SBS), styrene-ethylene-butylene-styrene copolymers (SEBS), styrene-isoprene-styrene copolymers (SIS). These can be employed either alone or in combination.

In a preferred embodiment the elastomer comprises EPM or EPDM.

Preferably said olefinic elastomer is blended with an ethylene homo- or co-polymer. Suitable ethylene homo- or co-polymers are polyethylene or copolymers of ethylene with an unsaturated comonomer such as an α-olefin, a vinyl ester, or an olefinically unsaturated acid or ester.

Examples of blends of an ethylene homo- or co-polymer and an olefinic elastomer that can suitably be employed for the intermediate layer iv) are described for instance in EP-A-32,804 or in EP-A-194,150, the content of which is incorporated herein by reference.

Preferably an ethylene copolymer is employed, such as ethylene-vinyl acetate copolymer, ethylene-methyl acrylate copolymer, ethylene-butyl acrylate copolymer, ethylene-acrylic acid copolymer, or ethylene-methacrylic acid copolymer. More preferably ethylene-vinyl acetate copolymers are used wherein the amount of vinyl acetate derived units is comprised from about 9 to about 40 % by weight, and preferably from about 19 to about 32 % by weight.

When the elastomeric olefin is blended with an ethylene homo- or co-polymer, such a blend typically comprises from about 1 to about 55 parts by weight of ethylene homo-or co-polymer and from about 25 to about 99 parts by weight of the elastomeric component; preferably such a blend comprises from about 10 to about 55 parts by weight of ethylene homo- or co-polymer and from about 25 to about 90 parts by weight of the elastomeric component; and even more preferably such a blend comprises from about 30 to about 55 parts by weight of ethylene homo- or co-polymer and from about 25 to about 60 parts by weight of the elastomeric component.

Such a blend may also comprise a processing oil, such as a normally liquid hydrocarbon processing oil, typically categorized as aromatic, naphthenic, or paraffinic process oil of a medium viscosity range.

When such a processing oil is used, it will preferably be used in an amount not higher than 20, preferably not higher than 15, and even more preferably not higher than 10 parts by weight. Preferably at least 5 parts by weight of processing oil are employed in the blend.

A preferred blend comprises from about 35 to about 55 parts by weight of ethylene-vinyl acetate copolymer, from about 25 to about 55 parts by weight of an EPM or EPDM elastomer and from about 5 to about 20 parts by weight of processing hydrocarbon oil.

A most preferred blend comprises from about 40 to about 50 parts by weight of ethylene-vinyl acetate copolymer, from about 35 to about 45 parts by weight of an EPM or EPDM elastomer and from about 5 to about 10 parts by weight of processing hydrocarbon oil.

The thickness of the intermediate layer(s) iv) may depend on the required overall thickness of the structure, the number of layers thereof, and whether the olefinic elastomer is blended or not with the ethylene homo- or copolymer. However, in order to get the advantages of softness provided by the use of the olefinic elastomer, the thickness of the intermediate layer iv) is at least about 8 µ, preferably at least about 15 µ, and even more preferably at least about 26 µ. In case there are two intermediate layers iv), the above values refer to the combined thickness of the two layers iv).

The overall thickness of a film suitable for the manufacture of ostomy pouches is typically comprised between about 50 and about 150 µ, and preferably comprised between about 60 and about 120 µ.

Opacifying additives and pigments can be included in one or more layers of the film, preferably the exterior layers to obtain an opaque, white or coloured, film.

Other additives, such as for instance antiblock and slip agents, UV absorbers, stabilisers, anti-oxidant agents, etc., can also be included as known in the art.

The film according to the present invention can be obtained by means of conventional technology, for example by hot-blown, i.e. by coextrusion of the multilayer film through a circular die with multiple concentric slots, using the insufflation of a bubble of cold air while the polymers are still in the molten phase, or by cast coextrusion, either through a round or a flat die, according to methods known *per se* in this field. In this latter case, alternatively the multi-layer film may also be prepared by extrusion coating.

If desired, the end film thus obtained can be embossed in order to further improve the patient's comfort.

While the intermediate layer iv) will provide the overall structure with the desired quietness, pliability and softness features, resistance to wear, abrasion, and puncturing, and heat-sealability can be optimised by suitably selecting the outer layers ii) and iii).

The examples which follow, only purport to illustrate the invention and not to restrict its field of application.

The following abbreviations have been used
- EVA1 =: ethylene-vinyl acetate copolymer with 18 % VA, MFI = 0.7 g/10' (as determined by ASTM D-1238) marketed by DuPont as ELVAX™ 3165 containing about 0.5 % erucamide and about 0.5 % silica;
- EVA2 =: ethylene-vinyl acetate copolymer with 28 % VA, MFI = 4.0 g/10' marketed by Elf Atochem as EVATANE™2803;
- EMA =: ethylene-methyl acrylate copolymer with 17 % MA, MFI = 2.4 g/10' marketed by Gulf as POLY-ETH 2255;
- EBA =: ethylene-butyl acrylate copolymer with 17 % BA, MFI = 2.2 g/10' marketed by Borealis as NCPE 5440;
- CPE1 =: a blend of 70 % by weight of chlorinated polyethylene (chlorine content 40 % by weight) sold by Dow as TYRIN BH 9000 and 30 % by weight of EVA1;
- CPE2 =: a blend of 70 % by weight of chlorinated polyethylene (chlorine content 40 % by weight) sold by Dow as TYRIN BH 9000, 25 % by weight of EVA1 and 5 % by weight of process hydrocarbon oil;
- PVDC =: VDC-VC copolymer with 4 % epoxidised soya bean oil and minor amounts of stabilisers;
- EVOH =: ethylene-vinyl alcohol copolymer with 44 % by mole of ethylene marketed by Marubeni as EVAL™ EP-E105A;
- EPM1 =: ethylene-propylene elastomer commercialised by Enichem as DUTRAL™ CO 034 PL with approximately 28 % by weight of propylene and Mooney viscosity [ML (1 + 4) at 100°C] of about 40;
- EPM2 =: ethylene-propylene elastomer commercialised by Enichem as DUTRAL™ CO 038 PL with approximately 27 % by weight of propylene and Mooney viscosity [ML (1 + 4) at 100°C] of about 60;
- EPM3 =: ethylene-propylene elastomer commercialised by Enichem as DUTRAL™ CO 053 with approximately 45 % by weight of propylene and Mooney viscosity [ ML (1 + 4) at 100°C] of about 43;
- SEBS =: styrene-ethylene-butylene-styrene block copolymer with a styrene content of approximately 28 % by weight, marketed by Shell as CARIFLEX™ TR 1102;
- BL1 =: a blend of about 45 % by weight of ethylene-vinyl acetate copolymer with 26 % VA, about 40 % of ethylene-propylene rubber, about 8 % of process hydrocarbon oil, about 5.5 % of calcium carbonate and about 1 % of slip agent, marketed by Exxon Chemical as Escorene™ EA 001;
- BL2 =: blend of 85 % EPM3 and 15 % EVA1;
- BL3 =: blend of 70 % EPM3 and 30 % EVA1;
- BL4 =: blend of 55 % EPM3 and 45 % EVA1;
- BL5 =: blend of 70 % EPM3, 15 % HDPE, and 15 % EVA1;
- BL6 =: blend of 70 % EPM3, 10 % HDPE, and 20% EVA1;

### Examples 1 to 15

5-layer symmetrical films having the general structure
A/B/C/B/A
wherein the composition and thickness of the A, B, and C layers are as indicated in Table 1 below, are prepared by the hot blown process.

The films are then converted into ostomy pouches by conventional heat-sealing.

**TABLE 1**

| Ex. no. | A | B | C |
|---|---|---|---|
| 1 | EVA1 (16 µ) | BL1 (16.5 µ) | PVDC (10 µ) |
| 2 | EVA1 (20 µ) | SEBS (25 µ) | PVDC (10 µ) |
| 3 | EVA1 (20 µ) | 80 % SEBS + 20 % EVA2 (25 µ) | PVDC (10 µ) |
| 4 | EVA1 (20 µ) | EPM1 (25 µ) | PVDC (10 µ) |
| 5 | EVA1 (20 µ) | EPM2 (25 µ) | PVDC (10 µ) |
| 6 | EVA1 (20 µ) | BL2 (25 µ) | PVDC (10 µ) |
| 7 | EVA1 (20 µ) | BL3 (25 µ) | PVDC (10 µ) |
| 8 | EVA1 (20 µ) | BL4 (25 µ) | PVDC (10 µ) |
| 9 | EVA1 (20 µ) | BL5 (25 µ) | PVDC (10 µ) |
| 10 | EVA1 (20 µ) | BL6 (25 µ) | PVDC (10 µ) |
| 11 | EBA (16 µ) | BL1 (16.5 µ) | PVDC (10 µ) |
| 12 | EMA (20 µ) | SEBS (25 µ) | PVDC (10 µ) |
| 13 | 50 % EVA1+ 50 % EBA (20 µ) | BL1 (25 µ) | PVDC (10 µ) |
| 14 | CPE1 (20 µ) | EPM1 (25 µ) | PVDC (10 µ) |
| 15 | CPE2 (20 µ) | EPM1 (25 µ) | PVDC (10 µ) |

### Example 16

A tan coloured opaque 5-layer symmetrical film, having the same general structure as in Example 1 with the only difference that the outer layers A differ from those of Example 1 for the presence of about 4 % Calcium carbonate, 2 % Titanium dioxide and 0.5 % of tan pigments, is prepared by hot blown and converted into ostomy pouches.

### Examples 17 and 18

4-layer films having the general structure
A/B/C/A
wherein the composition and thickness of the A, B, and C layers are as indicated in Table 2 below, are prepared by the hot blown process and converted into ostomy pouches.

**TABLE 2**

| Ex. no. | A | B | C |
|---|---|---|---|
| 17 | EVA1 (20 µ) | BL1 (50 µ) | PVDC (10 µ) |
| 18 | EVA1 (10 µ) | BL1 (70 µ) | PVDC (10 µ) |

### Examples 19 and 20

7-layer symmetrical films having the general structure
A/B/D/C/D/B/A
wherein the composition and thickness of the A, B, D, and C layers are as indicated in Table 3 below, are prepared by the hot blown process and converted into ostomy pouches.

**TABLE 3**

| Ex. no. | A | B | D | C |
|---|---|---|---|---|
| 19 | EVA1 (15 µ) | BL1 (25 µ) | TIE1 (5 µ) | PVDC (10 µ) |
| 20 | EVA1 (15 µ) | BL1 (25 µ) | TIE1 (5 µ) | EVOH (10 µ) |

### Examples 21 to 26

5-layer asymmetrical films having the general structure
A/B/C/B/D
wherein the composition and thickness of the A, B, C, and D layers are as indicated in Table 4 below, are prepared by the hot blown process and converted into ostomy pouches.

**TABLE 4**

| Ex. no. | A | B | C | D |
|---|---|---|---|---|
| 21 | EVA1 (15 µ) | BL1 (30 µ) | PVDC (10 µ) | EBA (15 µ) |
| 22 | EVA1 (20 µ) | EPM3 (25 µ) | PVDC (10 µ) | 50% EMA + 50% EBA (20 µ) |
| 23 | EVA1 (15 µ) | BL1 (30 µ) | PVDC (10 µ) | CPE1 (15 µ) |
| 24 | EVA1 (20 µ) | BL1 (35 µ) | PVDC (10 µ) | CPE2 (20 µ) |
| 25 | EVA1 (10 µ) | BL1 (35 µ) | PVDC (10 µ) | 50% EVA1 + 50% EBA (10 µ) |
| 26 | EVA1 (20 µ) | BL1 (30 µ) | PVDC (10 µ) | EMA (20 µ) |

Quietness or absence of noise of a representative example of the film according to the present invention has been evaluated by a noise emission test specifically designed for ostomy pouches.

Said test is based on the simulation of the stresses applied by ostomates' body on ostomy pouches during everyday activities.

Stress simulation is obtained by applying to the pouch an axial stroke simultaneously with a 180° rotation at the frequency of 30 cycles/min, and the noise generated by the ostomy pouch under such stresses is measured in the range of frequencies that can be perceived by a human ear (i.e., from 200 to 25,000 Hz).

In the actual practice, the test is run by placing the pouch to be tested and the twisting device in a anechoic chamber equipped with a microphone that records the noise, analyses it and plots it in a graph acoustical pressure (dB) versus noise frequency (Hz).

In the test, the overall noise emission in the frequency range considered is calculated as the spectrum integral value and the "background noise", i.e. the noise emission of the empty twisting device, is subtracted therefrom. Ten pouches are tested for each formulation and the arithmetic average of the adjusted readings is then calculated and used to express the film noise.

In this test the pouch of Example 1 gave a noise value of 45.0 dB while the noise value of a similar pouch made from a Comparative Film A, differing from the film of Example 1 only in the composition of the B layers that consists of EVA2, was 46.1 dB.

The added features of resistance to wear, abrasion, and puncturing, as well as gas and odour barrier capabilities, qualify the films according to the present invention for use in the manufacture of containers, e.g. bags and pouches, for medical applications, especially for draining and collecting excretion products from stomized patients.

## Claims

1. A multi-layer gas- and odour-barrier film comprising
i) a core gas- and odour-barrier layer
ii) a first exterior sealing layer
iii) a second exterior abuse and skin contact layer, and
iv) at least one intermediate layer positioned between the core barrier layer and at least one of the exterior layers ii) and iii), comprising an olefinic elastomer optionally blended with an ethylene homopolymer and/or copolymer.

2. A film as in claim 1 wherein the first exterior sealing layer ii) is a polyolefin layer.

3. A film as in claim 1 wherein the intermediate layer iv) comprises a blend of an olefinic elastomer with an ethylene homopolymer and/or copolymer.

4. A film as in claim 1 comprising two intermediate layers iv), each one being positioned between the core gas-barrier layer i) and either one of the exterior layers ii) and iii).

5. A film as in claim 4 which is symmetrical.

6. A film as in claim 3 wherein the blend of intermediate layer iv) comprises from about 1 to about 55 parts by weight of ethylene homo- and/or co-polymer and from about 25 to about 99 parts by weight of the elastomeric component.

7. A film as in claim 6 wherein the blend of intermediate layer iv) comprises from about 10 to about 55 parts by weight of ethylene homo- and/or co-polymer and from about 25 to about 90 parts by weight of the elastomeric component.

8. A film as in claim 7 wherein the blend of intermediate layer iv) comprises from about 30 to about 55 parts by weight of ethylene homo- and/or co-polymer and from about 25 to about 60 parts by weight of the elastomeric component.

9. A film as in claim 8 wherein the blend of intermediate layer iv) further comprise from about 5 to about 20 parts by weight of a normally liquid hydrocarbon processing oil.

10. A film as in claim 9 wherein the normally liquid hydrocarbon processing oil is present in an amount of from about 5 to about 10 parts by weight.

11. A film as in claim 9 or 10 wherein the hydrocarbon processing oil is an aromatic, naphthenic, or paraffinic process oil of a medium viscosity range.

12. A film as in claim 3 wherein the ethylene homo- and/or copolymer of the blend of intermediate layer iv) is an ethylene-vinyl acetate copolymer.

13. A film as in claim 3 wherein the olefinic elastomer of intermediate layer iv) is an ethylene-propylene elastomer.

14. A film as in claim 12 wherein the blend of intermediate layer iv) comprises from about 40 to about 50 parts by weight of ethylene-vinyl acetate copolymer, from about 35 to about 45 parts by weight of an EPM or EPDM and from about 5 to about 10 parts by weight of processing hydrocarbon oil.

15. A film as in claim 1 wherein the outer layer iii) comprise one or more resins selected from the group consisting of thermoplastic polyolefins.

16. A film as in claim 15 wherein the thermoplastic polyolefins are selected from the group consisting of heterogeneous ethylene-α-olefins with a density below 0.925 g/cc, preferably below 0.915 g/cc, homogeneous ethylene-α-olefins with a density below 0.920 g/cc preferably below 0.910 g/cc, ethylene-vinyl acetate copolymers, ethylene-alkyl acrylate copolymers, e.g. ethylene-methyl acrylate copolymers, and ethylene-butyl acrylate copolymers, and chlorinated polyolefins.

17. A film as in any of the preceding claims wherein the core barrier layer i) comprises ethylene-vinyl alcohol copolymer or PVDC.

18. A film as in claim 17 wherein the core barrier layer i) comprises PVDC.

19. A bag or container intended in particular for use in draining and collecting excretion products from patients whose excretive apparatus has been reconstructed and/or deviated, characterised in that it is formed from a film according to any one of preceding claims 1 to 18.
